(19) Europäisches Patentamt
European Patent Office
Office européen des brevets

(11) **EP 3 044 199 B1**

(12) **EUROPÄISCHE PATENTSCHRIFT**

(45) Veröffentlichungstag und Bekanntmachung des Hinweises auf die Patenterteilung:
**28.07.2021 Patentblatt 2021/30**

(21) Anmeldenummer: **14758860.2**

(22) Anmeldetag: **02.09.2014**

(51) Int Cl.:
***C07C 57/04*** *(2006.01)*

(86) Internationale Anmeldenummer:
**PCT/EP2014/068561**

(87) Internationale Veröffentlichungsnummer:
**WO 2015/036278 (19.03.2015 Gazette 2015/11)**

(54) **VERFAHREN ZUR DEHYDRATISIERUNG VON 3-HYDROXYPROPIONSÄURE ZU ACRYLSÄURE**

METHOD FOR THE DEHYDRATION OF 3-HYDROXYPROPANOIC ACID TO FORM ACRYLIC ACID

PROCÉDÉ DE DÉSHYDRATATION DE L'ACIDE 3-HYDROXYPROPIONIQUE POUR FORMER DE L'ACIDE ACRYLIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.09.2013 EP 13184095**

(43) Veröffentlichungstag der Anmeldung:
**20.07.2016 Patentblatt 2016/29**

(73) Patentinhaber: **BASF SE**
**67056 Ludwigshafen am Rhein (DE)**

(72) Erfinder:
• **BLASCHKE, Tim**
**70173 Stuttgart (DE)**
• **LANG, Ortmund**
**66909 Quirnbach (DE)**
• **WÖRZ, Nicolai Tonio**
**64293 Darmstadt (DE)**
• **RAITH, Christian**
**68167 Mannheim (DE)**
• **HARTMANN, Marco**
**76744 Wörth (DE)**
• **ZAJACZKOWSKI-FISCHER, Marta**
**67141 Neuhofen (DE)**

(56) Entgegenhaltungen:
**WO-A1-2005/095320     WO-A1-2007/106099
WO-A1-2007/106099     WO-A1-2008/023039
US-A1- 2007 219 391**

**Beschreibung**

Die Erfindung betrifft ein Verfahren zur Dehydratisierung von wässriger 3-Hydroxypropionsäure zu Acrylsäure in flüssiger Phase, wobei wässrige Acrylsäure kontinuierlich aus der flüssigen Phase entfernt wird und die flüssige Phase ein inertes organisches Lösungsmittel 1 enthält.

Acrylsäure ist wegen ihrer sehr reaktiven Doppelbindung sowie ihrer Carbonsäure-Gruppe ein wertvolles Monomer zur Herstellung von Polymeren, z.B. wasserabsorbierende Polymerpartikel, Bindemittel für wässrige Dispersionsfarben und in wässrigem Lösungsmittel dispergierte Klebstoffe.

Wasserabsorbierende Polymerpartikel werden zur Herstellung von Windeln, Tampons, Damenbinden und anderen Hygieneartikeln, aber auch als wasserzurückhaltende Mittel im landwirtschaftlichen Gartenbau verwendet. Die wasserabsorbierenden Polymerpartikel werden auch als Superabsorber bezeichnet.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Acrylsäure wird großtechnisch nahezu ausschließlich aus fossilen Rohstoffen hergestellt. Dies wird von den Konsumenten der Hygieneartikel als nachteilig angesehen. Es besteht daher ein Bedarf die in den Hygieneartikeln eingesetzten wasserabsorbierenden Polymerpartikel aus nachwachsenden Rohstoffen zu erzeugen.

Ein möglicher Weg ist die fermentative Herstellung von 3-Hydroxypropionsäure und deren Umsetzung zu Acrylsäure. Die Herstellung von 3-Hydroxypropionsäure durch Fermentation wird beispielsweise in WO 2012/074818 A2 beschrieben.

Die Dehydratisierung von 3-Hydroxypropionsäure in der Gasphase wird in US 7,538,247 erwähnt.

Die Dehydratisierung von 3-Hydroxypropionsäure in flüssiger Phase wird beispielsweise in WO 2006/092271 A2, WO 2008/023039 A1, JP 2010-180171, EP 2 565 211 A1 und EP 2 565 212 A1 erwähnt. Die Dehydratisierung von 3-Hydroxypropionsäure wird in WO2007/106099, US2007/219391 und WO2005/095320 erwähnt.

Aufgabe der vorliegenden Erfindung war die Bereitstellung eines verbesserten Verfahrens zur Herstellung von Acrylsäure auf Basis nachwachsender Rohstoffe.

Gelöst wurde die Aufgabe durch ein Verfahren zur kontinuierlichen Dehydratisierung von wässriger 3-Hydroxypropionsäure zu wässriger Acrylsäure in flüssiger Phase, wobei die flüssige Phase eine Temperatur von 120 bis 250°C aufweist, wässrige 3-Hydroxypropionsäure kontinuierlich der flüssigen Phase zugeführt wird und die wässrige Acrylsäure kontinuierlich aus der flüssigen Phase entfernt wird, dadurch gekennzeichnet, dass die flüssige Phase von 5 bis 95 Gew.-% eines inerten organischen Lösungsmittels 1 enthält.

Die flüssige Phase weist eine Temperatur von vorzugsweise 130 bis 220°C, besonders bevorzugt von 140 bis 200°C, ganz besonders bevorzugt von 150 bis 180°C, auf.

Das inerte organische Lösungsmittel 1 hat eine Löslichkeit in Wasser bei 23°C von vorzugsweise weniger al 5 g pro 100 ml Wasser, besonders bevorzugt weniger als 1 g pro 100 ml Wasser, ganz besonders bevorzugt von weniger als 0,2 g pro 100 ml Wasser.

Die im erfindungsgemäßen Verfahren eingesetzte wässrige 3-Hydroxypropionsäure enthält vorzugsweise mindestens 10 Gew.-%, besonders bevorzugt mindestens 20 Gew.-%, ganz besonders bevorzugt mindestens 30 Gew.-%, Wasser.

Die flüssige Phase enthält vorzugsweise von 10 bis 90 Gew.-%, besonders bevorzugt von 20 bis 80 Gew.-%, ganz besonders bevorzugt von 30 bis 60 Gew.-% des inerten organischen Lösungsmittels 1. In diesem Dokument werden die folgenden Druckeinheiten verwendet, die gemäss der folgenden Umrechnung in SI -Einheiten umgerechnet werden können: 1 mbar= 0,1KPa.

Der Siedepunkt des inerten organischen Lösungsmittels 1 liegt bei 1013 mbar im Bereich von vorzugsweise 200 bis 350°C, besonders bevorzugt von 250 bis 320°C, ganz besonders bevorzugt von 280 bis 300°C. Geeignete inerte organische Lösungsmittel 1 sind beispielsweise Phthalsäureester, wie Dimethylphthalat und Diethylphthalat, Isophthalsäureester, wie Dimethylisophthalat und Diethylisophthalat, Terephthalsäureester, wie Dimethylterephthalat und Diethylterephthalat, Alkansäuren, wie Nonansäure und Dekansäure, Biphenyl und/oder Diphenylether. Die bei der Dehydratisierung entstehende wässrige Acrylsäure wird vorzugsweise destillativ abgetrennt. Besonders geeignet sind hierfür Rektifikationskolonnen. Über die Auswahl der Trennstufen und des Rücklaufverhältnisses kann der Gehalt an 3-Hydroxypropionsäure im Destillat niedrig gehalten werden.

Vorteilhaft wird in der flüssigen Phase eine ausreichend lange Verweilzeit eingestellt, d.h. die Raum-Zeit-Ausbeute sollte nicht zu hoch sein. Zu niedrige Raum Zeit-Ausbeuten verteuern das Verfahren unnötig. Die Raum Zeit-Ausbeute beträgt vorzugsweise von 10 bis 150 kg/h Acrylsäure, besonders bevorzugt von 20 bis 100 kg/h Acrylsäure, ganz besonders bevorzugt 30 bis 80 kg/h Acrylsäure, jeweils pro kg flüssiger Phase. Die Raum-Zeit-Ausbeute ist der Quotient aus der pro Zeiteinheit abgetrennten Acrylsäure und dem Reaktorvolumen.

Die Verweilzeit in der flüssigen Phase beträgt vorzugsweise mindestens 10 Minuten, besonders bevorzugt mindestens 30 Minuten, ganz besonders bevorzugt mindestens 60 Minuten. Die Verweilzeit ist der Quotient aus Menge an flüssiger Phase in der Dehydratisierung und Zulaufmenge.

Das Verhältnis von oligomerer 3-Hydroxypropionsäure zu monomerer 3-Hydroxypropionsäure in der flüssigen Phase beträgt vorzugsweise mindestens 1:1, besonders bevorzugt mindestens 3:1, ganz besonders bevorzugt 5:1. Geringere Konzentrationen an monomerer 3-Hydroxypropionsäure in der flüssigen Phase, d.h. größere Verhältnisse von oligomerer 3-Hydroxypropionsäure zu monomerer 3-Hydroxypropionsäure, erleichtern die destillative Trennung von Acrylsäure und 3-Hydroxypropionsäure.

Das Verhältnis von oligomerer 3-Hydroxypropionsäure zu monomerer 3-Hydroxypropionsäure in der wässrigen 3-Hydroxypropionsäure (Zulauf) beträgt vorzugsweise mindestens 1:20, besonders bevorzugt mindestens 1:15, besonders bevorzugt mindestens 1:10, ganz besonders bevorzugt 1:5. Geringere Konzentrationen an monomerer 3-Hydroxypropionsäure in der wässrigen 3-Hydroxypropionsäure, d.h. größere Verhältnisse von oligomerer 3-Hydroxypropionsäure zu monomerer 3-Hydroxypropionsäure, erleichtern die Anreicherung an oligomerer 3-Hydroxypropionsäure in der flüssigen Phase.

Das Verhältnis von oligomerer 3-Hydroxypropionsäure zu monomerer 3-Hydroxypropionsäure im Sinne dieser Erfindung ist das Gewichtsverhältnis.

Vorteilhaft wird der flüssigen Phase ein Polymerisationsinhibitor 1 zugesetzt. Geeignete Polymerisationsinhibitoren 1 sind Phenothiazin, Hydrochinon und/oder Hydrochinonmonomethylether. Ganz besonders bevorzugt sind Phenothiazin und Hydrochinonmonomethylether. Bei Verwendung einer Rektifikationskolonne wird der Polymerisationsinhibitor 1 zumindest teilweise über den Rücklauf dosiert.

Vorteilhaft wird der wässrigen Acrylsäure ein Polymerisationsinhibitor 2 zugesetzt. Geeignete Polymerisationsinhibitoren 2 sind Phenothiazin, Hydrochinon und/oder Hydrochinonmonomethylether. Ganz besonders bevorzugt sind Phenothiazin und Hydrochinonmonomethylether.

Der vorliegenden Erfindung liegt die Erkenntnis zugrunde, dass die Polymerisationsneigung bei Verwendung eines inerten organischen Lösungsmittels 1 vermindert werden kann. Möglicherweise wird die Konzentration polymerisationsfähiger Acrylsäure hinreichend verdünnt.

Überraschenderweise wurde weiterhin festgestellt, dass sich unter den erfindungsgemäßen Reaktionsbedingungen aus 3-Hydroxypropionsäure bevorzugt oligomere 3-Hydroxypropionsäure bildet und sich oligomere 3-Hydroxypropionsäure leicht zu Acrylsäure umsetzen lässt. Eine Vermeidung von oligomerer 3-Hydroxypropionsäure, wie in WO 2012/091114 A1 gefordert, ist daher unnötig.

Oligomere 3-Hydroxypropionsäure ist das Produkt aus mindestens zwei Molekülen 3-Hydroxypropionsäure. Dabei werden die Moleküle untereinander durch Veresterung der Carboxyl-Gruppe des einen Moleküls mit der Hydroxyl-Gruppe des anderen Moleküls miteinander verbunden.

Oligomere Acrylsäure ist das Produkt aus mindestens zwei Molekülen Acrylsäure. Dabei werden die Moleküle untereinander durch Michael-Addition der Carboxyl-Gruppe des einen Moleküls mit der ethylenischen Doppelbindung des anderen Moleküls miteinander verbunden.

Im Folgenden wird das erfindungsgemäße Verfahren beschrieben:

Herstellung der 3-Hydroxypropionsäure

In dem erfindungsgemäßen Verfahren wird vorzugsweise durch Fermentation erzeugte wässrige 3-Hydroxypropionsäure eingesetzt. Ein derartiges Verfahren wird beispielsweise in WO 02/090312 A1 offenbart.

Die so erzeugte wässrige 3-Hydroxypropionsäure enthält üblicherweise neben Wasser im Wesentlichen folgende Bestandteile:

35 bis 70 Gew.-% 3-Hydroxypropionsäure.
0 bis 20 Gew.-% oligomere 3-Hydroxypropionsäure,
0 bis 10 Gew.-% Acrylsäure,
0 bis 1 Gew.-% oligomere Acrylsäure
0,01 bis 0,1 Gew.-% Glykolsäure,
0,01 bis 0,1 Gew.-% 2-Hydroxypropionsäure,
0,005 bis 0,05 Gew.-% Ameisensäure,
0,005 bis 0,05 Gew.-% Essigsäure,
0,005 bis 0,05 Gew.-% Bernsteinsäure,
0,005 bis 0,05 Gew.-% Fumarsäure,
0,0001 bis 0,01 Gew.-% Formaldehyd,
0,0001 bis 0,01 Gew.-% Acetaldehyd,
0,0001 bis 0,01 Gew.-% Methanol und
0,0001 bis 0,01 Gew.-% Ethanol

Herstellung von Acrylsäure

Die Dehydratisierung von 3-Hydroxypropionsäure wird in flüssiger Phase bei einer Temperatur von 120 bis 300°C, vorzugsweise von150 bis 250°C, besonders bevorzugt von 170 bis 230°C, ganz besonders bevorzugt von 180 bis 220°C, durchgeführt. Der Druck unterliegt keinen Beschränkungen. Ein geringer Unterdruck ist aus Sicherheitsgründen vorteilhaft.

Die flüssige Phase enthält vorzugsweise einen Polymerisationsinhibitor 1. Geeignete Polymerisationsinhibitoren 1 sind Phenothiazin, Hydrochinon und/oder Hydrochinonmonomethylether. Ganz besonders bevorzugt sind Phenothiazin und Hydrochinonmonomethylether. Die flüssige Phase enthält vorzugsweise von 0,001 bis 5 Gew.-%, besonders bevorzugt von 0,01 bis 2 Gew.-%, ganz besonders bevorzugt von 0,1 bis 1 Gew.-% des Polymerisationsinhibitors 1. Vorteilhaft wird zusätzlich ein Sauerstoff-haltiges Gas zur Polymerisationsinhibierung eingesetzt. Besonders geeignet sind hierzu Luft/Stickstoff-Gemische mit einem Sauerstoffgehalt von 6 Vol.-% (Magerluft).

Die flüssige Phase enthält 5 bis 95 Gew.-%, vorzugsweise von 10 bis 90 Gew.-%, besonders bevorzugt von 20 bis 80 Gew.-%, ganz besonders bevorzugt von 30 bis 60 Gew.-% des inerten organischen Lösungsmittels 1.

Der Siedepunkt des inerten organischen Lösungsmittels 1 liegt bei 1013 mbar im Bereich von vorzugsweise 200 bis 350°C, besonders bevorzugt von 250 bis 320°C, ganz besonders bevorzugt von 280 bis 300°C. Geeignete inerte organische Lösungsmittel 1 sind beispielsweise Dimethylphthalat, Diethylphthalat, Dimethylisophthalat, Diethylisophthalat, Dimethylterephthalat, Diethylterephthalat, Alkansäuren, wie Nonansäure und Dekansäure, Biphenyl und/oder Diphenylether.

Das inerte organische Lösungsmittel 1 hat eine Löslichkeit in Wasser bei 23°C von vorzugsweise weniger al 5 g pro 100 ml Wasser, besonders bevorzugt weniger als 1 g pro 100 ml Wasser, ganz besonders bevorzugt von weniger als 0,2 g pro 100 ml Wasser.

Die Dehydratisierung kann basisch oder sauer katalysiert werden. Geeignete basische Katalysatoren sind hochsiedende tertiäre Amine, wie Pentamethyldiethylentriamin. Geeignete saure Katalysatoren sind hochsiedende anorganische oder organische Säuren, wie Phosphorsäure und Dodecylbenzolsulfonsäure. Hochsiedend bedeutet hierbei ein Siedepunkt bei 1013 mbar von vorzugsweise mindestens 160°C, besonders bevorzugt mindestens 180°C, ganz besonders bevorzugt mindestens 190°C.

Die Mengen an Katalysator in der flüssigen Phase beträgt vorzugsweise von 1 bis 60 Gew.-%, besonders bevorzugt von 2 bis 40 Gew.-%, ganz besonders bevorzugt von 5 bis 20 Gew.-%.

Die Dehydratisierung wird kontinuierlich durchgeführt. Die Wärmezufuhr kann über innen- und/oder außenliegende Wärmetauscher herkömmlicher Bauart und/oder über Doppelwandheizung erfolgen (als Wärmeträger wird vorteilhaft Wasserdampf verwendet). Vorzugsweise erfolgt sie über außenliegende Umlaufverdampfer mit Natur- oder Zwangsumlauf. Besonders bevorzugt werden außenliegende Umlaufverdampfer mit Zwangsumlauf eingesetzt. Derartige Verdampfer werden in EP 0 854 129 A1 beschrieben. Der Einsatz mehrerer Verdampfer, in Reihe oder parallel geschaltet, ist möglich.

Wird ein Sauerstoff-haltiges Gas zur Polymerisationsinhibierung eingesetzt, so wird dies vorzugsweise unterhalb des Verdampfers zugeführt.

Der Zulauf zum Reaktor wird vorzugweise auf eine Temperatur von 30 bis 100°C, besonders bevorzugt von 40 bis 95°C, ganz besonders von 50 bis 90°C, vorgewärmt.

Das bei der Dehydratisierung entstehende Wasser/Acrylsäure-Gemisch wird vorzugsweise destillativ abgetrennt, besonders bevorzugt mittels einer Rektifikationskolonne 1.

Die Rektifikationskolonne 1 ist von an sich bekannter Bauart und weist die üblichen Einbauten auf. Als Kolonneneinbauten kommen prinzipiell alle gängigen Einbauten in Betracht, beispielsweise Böden, Packungen und/oder Schüttungen. Unter den Böden sind Glockenböden, Siebböden, Ventilböden, Thormannböden und/oder Dual-Flow-Böden bevorzugt, unter den Schüttungen sind solche mit Ringen, Wendeln, Sattelkörpern, Raschig-, Intos- oder Pall-Ringen, Berl- oder Intalox-Sätteln oder Geflechten bevorzugt. Besonders bevorzugt sind Dual-Flow-Böden.

In der Regel sind 3 bis 10 theoretische Böden in der Rektifikationskolonne 1 ausreichend. Die Rektifikation wird üblicherweise bei leicht vermindertem Druck durchgeführt, vorzugsweise bei einem Kopfdruck von 900 bis 980 mbar. Der Sumpfdruck ergibt sich aus dem Kopfdruck, der Anzahl und der Art der Kolonneneinbauten sowie den fluidynamischen Erfordernissen der Rektifikation.

Die Rektifikationskolonne 1 ist üblicherweise aus austenitischem Stahl gefertigt, vorzugweise aus dem Werkstoff 1.4571 (nach DIN EN 10020).

Die Abkühlung der am Kopf der Rektifikationskolonne 1 abgetrennten wässrigen Acrylsäure kann indirekt, beispielsweise durch Wärmetauscher, die dem Fachmann an sich bekannt sind und keiner besonderen Beschränkung unterliegen, oder direkt, beispielsweise durch einen Quench, erfolgen. Vorzugsweise erfolgt sie durch direkte Kühlung. Dazu wird bereits kondensierte wässrige Acrylsäure mittels eines geeigneten Wärmetauschers gekühlt und die gekühlte Flüssigkeit oberhalb der Abnahmestelle im Brüden versprüht. Dieses Versprühen kann in einem getrennten Apparat oder in der Rektifikationseinheit selbst erfolgen. Beim Versprühen in der Rektifikationseinheit ist die Entnahmestelle der wässrigen Acrylsäure vorteilhafterweise als Fangboden ausgebildet. Durch Einbauten, die die Durchmischung der gekühlten wässrigen Acrylsäure mit dem Brüden verbessern, kann die Wirkung der direkten Kühlung gesteigert werden. Hierzu kommen prinzipiell alle gängigen Einbauten in Betracht, beispielsweise Böden, Packungen und/oder Schüttungen. Unter den Böden sind Glockenböden, Siebböden, Ventilböden, Thormannböden und/oder Dual-Flow-Böden bevorzugt. Unter den Schüttungen sind solche mit Ringen, Wendeln, Sattelkörpern, Raschig-, Intos- oder Pall-Ringen, Berl- oder Intalox-Sätteln oder Geflechten bevorzugt. Besonders bevorzugt sind Dual-Flow-Böden. In der Regel sind hier 2 bis 5 theoretische Böden ausreichend. Diese Böden sind bei den bisher erfolgten Angaben zur Anzahl der theoretischen Böden der Rektifikationskolone 1 nicht berücksichtigt. Die direkte Kondensation der wässrigen Acrylsäure kann auch mehrstufig ausgeführt sein, mit nach oben abnehmender Temperatur.

Bei Verwendung eines wasserunlöslichen inerten organischen Lösungsmittels 1 wird das kondensierte Destillat der Rektifikationskolonne 1 mittels eines Phasenscheiders getrennt. Die organische Phase kann in die Rektifikationskolonne 1 zurückgeführt werden, beispielsweise in den Sumpf. Die wässrige Phase kann ebenfalls teilweise in die Rektifikationskolonne 1 zurückgeführt werden, beispielsweise als Rücklauf und zur direkten Kühlung des Brüdens.

Die über Kopf der Rektifikationskolonne 1 abgetrennte wässrige Acrylsäure enthält üblicherweise neben Wasser und Spuren des inerten organischen Lösungsmittels 1 im Wesentlichen folgende Bestandteile:

0 bis 0,001 Gew.-% 3-Hydroxypropionsäure.
0 bis 0,001 Gew.-% oligomere 3-Hydroxypropionsäure,
20 bis 80 Gew.-% Acrylsäure,
0 bis 0,001 Gew.-% oligomere Acrylsäure
0,001 bis 1 Gew.-% Glykolsäure,
0 bis 0,001 Gew.-% 2-Hydroxypropionsäure,
0,001 bis 1 Gew.-% Ameisensäure,
0,001 bis 1 Gew.-% Essigsäure,
0 bis 0,001 Gew.-% Bernsteinsäure,
0 bis 0,001 Gew.-% Fumarsäure,
0,0001 bis 0,05 Gew.-% Formaldehyd,
0,0001 bis 0,05 Gew.-% Acetaldehyd,
0,0001 bis 0,05 Gew.-% Methanol und
0,0001 bis 0,05 Gew.-% Ethanol

Ein Teil der entnommenen wässrigen Acrylsäure, vorzugsweise 10 bis 40 Gew.-% bezogen auf die Gesamtmenge an Destillat, wird als Rücklauf verwendet, der Rest der wässrigen Acrylsäure wird ausgeschleust.

Die bei der Dehydratisierung erhaltene wässrige Acrylsäure kann in einer Extraktionskolonne aufgearbeitet werden.

Vorteilhaft wird eine Teilmenge der flüssigen Phase dem Reaktor entnommen, mit Wasser gewaschen und nach Phasentrennung zurückgeführt. Die so erhalten wässrige Phase kann verworfen oder nach weiteren Reinigungsschritten zusammen mit der am Kopf der Rektifikationskolonne 1 entnommenen wässrigen Acrylsäure der Extraktionskolonne zugeführt werden.

Die Extraktionskolonne ist von an sich bekannter Bauart und kann die üblichen Einbauten aufweisen. Als Kolonneneinbauten kommen prinzipiell alle gängigen Einbauten in Betracht. Beispiele sind Böden, Packungen und/oder Schüttungen. Unter den Böden sind Siebböden und/oder Dual-Flow-Böden bevorzugt. Unter den Schüttungen sind solche mit Ringen, Wendeln, Sattelkörpern, Raschig-, Intos- oder Pall-Ringen, Berl- oder Intalox-Sätteln oder Geflechten bevorzugt. Besonders bevorzugt sind Dual-Flow-Böden. In der Regel sind hier 10 bis 25 theoretische Böden ausreichend.

Die Extraktionskolonne wird bei einer Temperatur von vorzugsweise 30 bis 70°C, besonders bevorzugt 40 bis 60°C, ganz besonders bevorzugt 45 bis 55°C, betrieben.

In der Extraktionskolonne wird Acrylsäure mittels eines inerten organischen Lösungsmittels 2 aus der wässrigen Phase extrahiert. Der Siedepunkt des inerten organischen Lösungsmittels 2 liegt bei 1013 mbar im Bereich von vorzugsweise 200 bis 350°C, besonders bevorzugt von 250 bis 320°C, ganz besonders bevorzugt von 280 bis 300°C. Geeignete inerte organische Lösungsmittel 2 sind beispielsweise Phthalsäureester, wie Dimethylphthalat und Diethylphthalat, Isophthalsäureester, wie Dimethylisophthalat und Diethylisophthalat, Terephthalsäureester, wie Dimethylterephthalat und Diethylterephthalat, Alkansäuren, wie Nonansäure und Dekansäure, Biphenyl und/oder Diphenylether.

Das inerte organische Lösungsmittel 2 hat eine Löslichkeit in Wasser bei 23°C von vorzugsweise weniger al 5 g pro 100 ml Wasser, besonders bevorzugt weniger als 1 g pro 100 ml Wasser, ganz besonders bevorzugt von weniger als 0,2 g pro 100 ml Wasser.

In einer besonders bevorzugten Ausführungsform der vorliegenden Erfindung wird das inerte organische Lösungsmittels 1 auch als inertes organisches Lösungsmittel 2 eingesetzt. In diesem Fall kann der Phasenscheider am Kopf der Rektifikationskolonne 1 entfallen.

Das Verhältnis von wässriger Phase (wässrige Acrylsäure) und organischer Phase (inertes organisches Lösungsmittel 2) beträgt vorzugsweise von 0,5:1 bis 1,5:1. Zur Aufrechterhaltung des Verhältnisses kann ein Teil des wässrigen Extrakts in die Extraktionskolonne zurückgeführt werden.

Das am Kopf der Extraktionskolonne abgetrennte wässrige Extrakt kann verworfen werden und enthält üblicherweise neben Wasser und Spuren der inerten organischen Lösungsmittel und ggf. des eingesetzten Katalysators im Wesentlichen folgende Bestandteile:

0,005 bis 0,1 Gew.-% 3-Hydroxypropionsäure.
0,05 bis 1 Gew.-% oligomere 3-Hydroxypropionsäure,
0,1 bis 2 Gew.-% Acrylsäure,
0,1 bis 2 Gew.-% oligomere Acrylsäure
0,01 bis 1 Gew.-% Glykolsäure,
0,01 bis 1 Gew.-% 2-Hydroxypropionsäure,
0,01 bis 0,2 Gew.-% Ameisensäure,
0,005 bis 0,1 Gew.-% Essigsäure,
0,01 bis 0,2 Gew.-% Bernsteinsäure,
0,01 bis 0,2 Gew.-% Fumarsäure,
0,002 Gew.-% Formaldehyd,
0 bis 0,001 Gew.-% Acetaldehyd,
0,0002 bis 0,01 Gew.-% Methanol und
0 bis 0,002 Gew.-% Ethanol

Das am Boden der Extraktionskolonne abgetrennte organische Extrakt enthält üblicherweise neben den inerten organischen Lösungsmitteln und ggf. des Katalysators im Wesentlichen folgende Bestandteile:

0,0005 bis 0,01 Gew.-% 3-Hydroxypropionsäure.
0,01 bis 1 Gew.-% oligomere 3-Hydroxypropionsäure,
1 bis 5 Gew.-% Wasser,
10 bis 35 Gew.-% Acrylsäure,
0,01 bis 1 Gew.-% oligomere Acrylsäure
0,005 bis 0,5 Gew.-% Glykolsäure,
0,005 bis 0,2 Gew.-% 2-Hydroxypropionsäure,
0,0001 bis 0,1 Gew.-% Ameisensäure,
0,001 bis 0,1 Gew.-% Essigsäure,
0,001 bis 0,05 Gew.-% Bernsteinsäure,
0,001 bis 0,1 Gew.-% Fumarsäure,
0,0001 bis 0,01 Gew.-% Formaldehyd,
0,0001 bis 0,005 Gew.-% Acetaldehyd,
0,0001 bis 0,005 Gew.-% Methanol und
0,0001 bis 0,01 Gew.-% Ethanol

Das bei der Extraktion erhaltene organische Extrakt kann in einer Rektifikationskolonne 2 aufgearbeitet werden.

Die Rektifikationskolonne 2 ist von an sich bekannter Bauart und weist die üblichen Einbauten auf. Als Kolonneneinbauten kommen prinzipiell alle gängigen Einbauten in Betracht, beispielsweise Böden, Packungen und/oder Schüttungen. Unter den Böden sind Glockenböden, Siebböden, Ventilböden, Thormannböden und/oder Dual-Flow-Böden bevorzugt, unter den Schüttungen sind solche mit Ringen, Wendeln, Sattelkörpern, Raschig-, Intos- oder Pall-Ringen, Berl- oder Intalox-Sätteln oder Geflechten bevorzugt. Besonders bevorzugt sind Dual-Flow-Böden.

In der Regel sind 10 bis 25 theoretische Böden in der Rektifikationseinheit ausreichend. Die Rektifikation wird üblicherweise bei vermindertem Druck durchgeführt, vorzugsweise bei einem Kopfdruck von 70 bis 140 mbar. Der Sumpfdruck ergibt sich aus dem Kopfdruck, der Anzahl und der Art der Kolonneneinbauten sowie den fluiddynamischen Erfordernissen der Rektifikation und beträgt vorzugsweise 200 bis 400 mbar.

Der Rücklauf der Rektifikationskolonne 2 enthält vorzugsweise einen Polymerisationsinhibitor 2. Geeignete Polymerisationsinhibitoren 2 sind Phenothiazin, Hydrochinon und/oder Hydrochinonmonomethylether. Ganz besonders bevorzugt ist Phenothiazin. Der Rücklauf enthält vorzugsweise von 0,0005 bis 1 Gew.-%, besonders bevorzugt von 0,002 bis 0,5 Gew.-%, ganz besonders bevorzugt von 0,01 bis 0,1 Gew.-% des Polymerisationsinhibitors 1. Vorteilhaft wird zusätzlich ein Sauerstoff-haltiges Gas zur Polymerisationsinhibierung eingesetzt. Besonders geeignet sind hierzu Luft/Stickstoff-Gemische mit einem Sauerstoffgehalt von 6 Vol.-% (Magerluft).

Die Rektifikationskolonne 2 ist üblicherweise aus austenitischem Stahl gefertigt, vorzugsweise aus dem Werkstoff

1.4571 (nach DIN EN 10020).

Der Zulauf in die Rektifikationskolonne 2 erfolgt zweckmäßig in ihrem unteren Bereich. Vorzugsweise erfolgt er 2 bis 5 theoretische Böden oberhalb des Sumpfes der Rektifikationskolonne 2. Die Zulauftemperatur beträgt vorzugsweise von 20 bis 200°C, besonders bevorzugt von 50 bis 180°C, ganz besonders bevorzugt von 80 bis 160°C.

Die Wärmezufuhr erfolgt über innen- und/oder außenliegende Wärmetauscher (Wärmeträger ist wieder vorzugsweise Wasserdampf) herkömmlicher Bauart und/oder über Doppelwandbeheizung. Vorzugsweise erfolgt sie über außenliegende Umlaufverdampfer mit Natur- oder Zwangsumlauf. Besonders bevorzugt sind außenliegende Umlaufverdampfer mit Zwangsumlauf. Derartige Verdampfer werden in EP 0 854 129 A1 beschrieben. Der Einsatz mehrerer Verdampfer, in Reihe oder parallel geschaltet, ist möglich. Vorzugsweise werden 2 bis 4 Verdampfer parallel betrieben. Die Sumpftemperatur der Rektifikationskolonne 2 beträgt typisch 180 bis 250°C, vorzugsweise 195 bis 235°C.

Wird ein Sauerstoff-haltiges Gas zur Polymerisationsinhibierung eingesetzt, so wird dies vorzugsweise unterhalb des Verdampfers zugeführt.

Die in den Sumpf der Rektifikationskolonne 2 anfallende Schwersiederfraktion enthält üblicherweise neben den inerten organischen Lösungsmitteln und ggf. des Katalysators im Wesentlichen folgende Bestandteile:

0,001 bis 0,05 Gew.-% 3-Hydroxypropionsäure.
0,01 bis 1 Gew.-% oligomere 3-Hydroxypropionsäure,
0 bis 0,0005 Gew.-% Wasser,
0,01 bis 1 Gew.-% Acrylsäure,
0,01 bis 1 Gew.-% oligomere Acrylsäure
0,005 bis 0,2 Gew.-% Glykolsäure,
0,005 bis 0,2 Gew.-% 2-Hydroxypropionsäure,
0 bis 0,0005 Gew.-% Ameisensäure,
0 bis 0,0005 Gew.-% Essigsäure,
0,001 bis 0,05 Gew.-% Bernsteinsäure,
0,001 bis 0,1 Gew.-% Fumarsäure,
0 bis 0,0005 Gew.-% Formaldehyd,
0 bis 0,0005 Gew.-% Acetaldehyd,
0 bis 0,0005 Gew.-% Methanol und
0 bis 0,0005 Gew.-% Ethanol

Die der Rektifikationskolonne 2 entnommene das inerte organische Lösungsmittel 2 enthaltende Sumpfflüssigkeit wird über einen Wärmetauscher in den Kopfbereich der Extraktionskolonne zurückgeführt. Die Sumpfflüssigkeit wird vorzugsweise über einen Feststoffabscheider (Zyklon) geführt und ggf. durch frisches inertes organisches Lösungsmittel 2 ergänzt,

Oberhalb des Zulaufs in die Rektifikationskolonne 2 wird über Seitenabzug eine Rohacrylsäure entnommen, vorzugsweise 8 bis 20 theoretische Böden oberhalb des Kolonnensumpfes. Die Entnahme der Rohacrylsäure erfolgt auf übliche Weise und unterliegt keiner Beschränkung. Geeignet ist die Entnahme über einen Fangboden, wobei der gesamte Rücklauf aufgefangen wird und ein Teil ausgeschleust und der andere Teil als Rücklauf unterhalb des Fangbodens verwendet wird, oder über einen Boden mit integrierter Abzugsmöglichkeit, vorzugsweise über einen Dual-Flow-Boden mit integrierter Abzugsmöglichkeit.

Die entnommene Rohacrylsäure enthält üblicherweise neben Acrylsäure im Wesentlichen folgende Bestandteile:

0 bis 0,0005 Gew.-% 3-Hydroxypropionsäure.
0 bis 0,0005 Gew.-% oligomere 3-Hydroxypropionsäure,
0 bis 5 Gew.-% Wasser,
0 bis 0,0005 Gew.-% oligomere Acrylsäure
0,001 bis 0,02 Gew.-% Glykolsäure,
0 bis 0,0005 Gew.-% 2-Hydroxypropionsäure,
0,005 bis 0,01 Gew.-% Ameisensäure,
0,01 bis 0,2 Gew.-% Essigsäure,
0 bis 0,0005 Gew.-% Bernsteinsäure,
0 bis 0,0005 Gew.-% Fumarsäure,
0 bis 0,0005 Gew.-% Formaldehyd,
0 bis 0,0005 Gew.-% Acetaldehyd,
0 bis 0,005 Gew.-% Methanol und
0 bis 0,005 Gew.-% Ethanol

Die entnommene Rohacrylsäure wird mittels eines Wärmetauschers abgekühlt (als Kühlmittel eignen sich z.B. Oberflächenwässer). Der Einsatz mehrerer Wärmetauscher, in Reihe oder parallel geschaltet, ist möglich. In den Wärmetauschern, die dem Fachmann an sich bekannt sind und keiner besonderen Beschränkung unterliegen, wird die Rohacrylsäure vorzugsweise auf 40 bis 90°C abgekühlt.

Die entnommene Rohacrylsäure wird ausgeschleust und teilweise als Lösungsmittel für den Polymerisationsinhibitor 2 verwendet.

Die Abkühlung des am Kopf der Rektifikationskolonne 2 abgetrennten Leichtsiederstroms kann indirekt, beispielsweise durch Wärmetauscher (als Kühlmittel können z.B. Oberflächenwässer verwendet werden), die dem Fachmann an sich bekannt sind und keiner besonderen Beschränkung unterliegen, oder direkt, beispielsweise durch einen Quench, erfolgen. Vorzugsweise erfolgt sie durch direkte Kühlung. Dazu wird bereits kondensierte Leichtsiederfraktion mittels eines geeigneten Wärmetauschers gekühlt und die gekühlte Flüssigkeit oberhalb der Abnahmestelle im Brüden versprüht. Dieses Versprühen kann in einem getrennten Apparat oder in der Rektifikationskolonne 2 selbst erfolgen. Beim Versprühen in der Rektifikationskolonne 2 ist die Entnahmestelle der Leichtsiederfraktion vorteilhafterweise als Fangboden ausgebildet. Durch Einbauten, die die Durchmischung der gekühlten Leichtsiederfraktion mit dem Brüden verbessern, kann die Wirkung der direkten Kühlung gesteigert werden. Hierzu kommen prinzipiell alle gängigen Einbauten in Betracht, beispielsweise Böden, Packungen und/oder Schüttungen. Unter den Böden sind Glockenböden, Siebböden, Ventilböden, Thormannböden und/oder Dual-Flow-Böden bevorzugt. Unter den Schüttungen sind solche mit Ringen, Wendeln, Sattelkörpern, Raschig-, Intos- oder Pall-Ringen, Berl- oder Intalox-Sätteln oder Geflechten bevorzugt. Besonders bevorzugt sind Dual-Flow-Böden. In der Regel sind hier 2 bis 5 theoretische Böden ausreichend. Diese Böden sind bei den bisher erfolgten Angaben zur Anzahl der theoretischen Böden der Rektifikationskolonne 2 nicht berücksichtigt. Die direkte Kondensation der Leichtsiederfraktion kann auch mehrstufig ausgeführt sein, mit nach oben abnehmender Temperatur.

Der über Kopf der Rektifikationskolonne 2 abgetrennte Leichtsiederstrom enthält üblicherweise neben Wasser im Wesentlichen folgende Bestandteile:

0 bis 0,0005 Gew.-% 3-Hydroxypropionsäure.
0 bis 0,0005 Gew.-% oligomere 3-Hydroxypropionsäure,
10 bis 60 Gew.-% Acrylsäure,
0 bis 0,0005 Gew.-% oligomere Acrylsäure
0 bis 0,0005 Gew.-% Glykolsäure,
0 bis 0,0005 Gew.-% 2-Hydroxypropionsäure,
0,01 bis 1 Gew.-% Ameisensäure,
0,01 bis 1 Gew.-% Essigsäure,
0 bis 0,0005 Gew.-% Bernsteinsäure,
0 bis 0,0005 Gew.-% Fumarsäure,
0,0005 bis 0,02 Gew.-% Formaldehyd,
0,0001 bis 0,01 Gew.-% Acetaldehyd,
0,0005 bis 0,02 Gew.-% Methanol und
0,005 bis 0,05 Gew.-% Ethanol

Ein Teil der als Leichtsiederfraktion entnommenen Flüssigkeit wird als Rücklauf verwendet, der Rest der Leichtsiederfraktion wird ausgeschleust und als wässrige Phase in die Extraktionskolonne zurückgeführt.

Die der Rektifikationskolonne 2 entnommene Rohacrylsäure kann direkt zur Herstellung wasserabsorbierender Polymerpartikel eingesetzt werden. Vorzugsweise wird die Rohacrylsäure durch Kristallisation weiter gereinigt. Die bei der Kristallisation anfallende Mutterlauge kann in die Rektifikationskolonne 2 zurückgeführt werden, vorzugsweise unterhalb der Abnahmestelle für die Rohacrylsäure.

Die Rohacrylsäure kann durch Schichtkristallisation, wie beispielsweise in EP 0 616 998 A1 beschrieben, oder durch Suspensionskristallisation, wie in DE 100 39 025 A1 beschrieben, gereinigt werden. Die Suspensionskristallisation ist bevorzugt. Die Kombination einer Suspensionskristalisation mit einer Waschkolone, wie in WO 2003/041832 A1 beschrieben, ist besonders bevorzugt.

Die so gereinigte Acrylsäure enthält üblicherweise neben Acrylsäure im Wesentlichen folgende Bestandteile:

<0,0001 Gew.-% 3-Hydroxypropionsäure.
<0,0001 Gew.-% oligomere 3-Hydroxypropionsäure,
0,01 bis 0,05 Gew.-% Wasser,
<0,0001 Gew.-% oligomere Acrylsäure
<0,0001 Gew.-% Glykolsäure,
<0,0001 Gew.-% 2-Hydroxypropionsäure,

<0,0001 Gew.-% Ameisensäure,
0,01 bis 0,05 Gew.-% Essigsäure,
<0,0001 Gew.-% Bernsteinsäure,
<0,0001 Gew.-% Fumarsäure,
<0,0001 Gew.-% Formaldehyd,
<0,0001 Gew.-% Acetaldehyd,
<0,0001 Gew.-% Methanol und
<0,0001 Gew.-% Ethanol

Die gemäß dem erfindungsgemäßen Verfahren hergestellte Acrylsäure kann zur Herstellung von Acrylsäureestern, wie Methylacrylat, Ethylacrylat, n-Butylacrylat und 2-Ethylhexylacrylat, und zur Herstellung von Polymeren, wie wasserabsorbierenden Polymerpartikeln verwendet werden.

Herstellung wasserabsorbierender Polymerpartikel

Wasserabsorbierender Polymerpartikel werden durch Polymerisation einer Monomerlösung oder -suspension, enthaltend

a) mindestens ein ethylenisch ungesättigtes, säuregruppentragendes Monomer, das zumindest teilweise neutralisiert sein kann, insbesondere teilneutralisierte Acrylsäure
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,

hergestellt und sind üblicherweise wasserunlöslich.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Weitere geeignete Monomere a) sind beispielsweise ethylenisch ungesättigte Sulfonsäuren, wie Styrolsulfonsäure und 2-Acrylamido-2-methylpropansulfonsäure (AMPS).

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/032962 A2 beschrieben.

Bevorzugte Vernetzer b) sind Pentaerythrittriallylether, Tetraallyloxyethan, Methylenbismethacrylamid, 15-fach ethoxyliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,05 bis 1,5 Gew.-%, besonders bevorzugt 0,1 bis 1 Gew.-

%, ganz besonders bevorzugt 0,2 bis 0,5 Gew.-%, jeweils bezogen auf Monomer a). Mit steigendem Vernetzergehalt sinkt die Zentrifugenretentionskapazität (CRC) und die Absorption unter einem Druck von 21,0 g/cm$^2$ durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; Deutschland) erhältlich.

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylacrylat.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit überschüssigem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die in Acrylsäure üblicherweise eingesetzten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Geeignete Reaktoren sind beispielsweise Knetreaktoren oder Bandreaktoren. Im Kneter wird das bei der Polymerisation einer wässrigen Monomerlösung oder -suspension entstehende Polymergel durch beispielsweise gegenläufige Rührwellen kontinuierlich zerkleinert, wie in WO 2001/038402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das in einem weiteren Verfahrensschritt zerkleinert werden muss, beispielsweise in einem Extruder oder Kneter.

Zur Verbesserung der Trocknungseigenschaften kann das mittels eines Kneters erhaltene zerkleinerte Polymergel zusätzlich extrudiert werden.

Es ist aber auch möglich eine wässrige Monomerlösung zu vertropfen und die erzeugten Tropfen in einem erwärmten Trägergasstrom zu polymerisieren. Hierbei können die Verfahrensschritte Polymerisation und Trocknung zusammengefasst werden, wie in WO 2008/040715 A2, WO 2008/052971 A1 und WO 2011/026876 A1 beschrieben.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 95 mol-%, besonders bevorzugt von 30 bis 80 mol-%, ganz besonders bevorzugt von 40 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Es ist aber auch möglich die Neutralisation nach der Polymerisation auf der Stufe des bei der Polymerisation entstehenden Polymergels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Wird das Polymergel zumindest teilweise nach der Polymerisation neutralisiert, so wird das Polymergel vorzugsweise mechanisch zerkleinert, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden.

Das Polymergel wird dann vorzugsweise mit einem Bandtrockner getrocknet bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt, wobei der Restfeuchtegehalt gemäß der von der EDANA empfohlenen Testmethode Nr. WSP 230.2-05 "Mass Loss Upon Heating" bestimmt wird. Bei einer zu hohen Restfeuchte weist das getrocknete Polymergel eine zu niedrige Glasübergangstemperatur $T_g$ auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Gels beträgt vor der Trocknung vorzugsweise von 25 bis 90 Gew.-%, besonders bevorzugt von 35 bis 70 Gew.-%, ganz besonders bevorzugt von 40 bis 60 Gew.-%. Wahlweise kann zur Trocknung aber auch ein Wirbelbetttrockner oder ein Schaufeltrockner verwendet werden.

Das getrocknete Polymergel wird hiernach gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion abgetrennten Polymerpartikel beträgt vorzugsweise mindestens 200 μm, besonders bevorzugt von 250 bis 600 μm, ganz besonders von 300 bis 500 μm. Die mittlere Partikelgröße der Produktfraktion kann mittels der von der EDANA empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Partikeln mit einer Partikelgröße von größer 150 μm beträgt vorzugsweise mindestens 90 Gew.-%, besonders bevorzugt mindesten 95 Gew.-%, ganz besonders bevorzugt mindestens 98 Gew.-%.

Polymerpartikel mit zu niedriger Partikelgröße senken die Permeabilität (SFC). Daher sollte der Anteil zu kleiner Polymerpartikel ("fines") niedrig sein.

Zu kleine Polymerpartikel werden daher üblicherweise abgetrennt und in das Verfahren rückgeführt. Dies geschieht vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Wird zur Polymerisation ein Knetreaktor verwendet, so werden die zu kleinen Polymerpartikel vorzugsweise während des letzten Drittels der Polymerisation zugesetzt.

Werden die zu kleinen Polymerpartikel sehr spät zugesetzt, beispielsweise erst in einem dem Polymerisationsreaktor nachgeschalteten Apparat, beispielsweise einem Extruder, so lassen sich die zu kleinen Polymerpartikel nur noch schwer in das erhaltene Polymergel einarbeiten. Unzureichend eingearbeitete zu kleine Polymerpartikel lösen sich aber während der Mahlung wieder von dem getrockneten Polymergel, werden beim Klassieren daher erneut abgetrennt und erhöhen die Menge rückzuführender zu kleiner Polymerpartikel.

Der Anteil an Partikeln mit einer Partikelgröße von höchstens 850 μm, beträgt vorzugsweise mindestens 90 Gew.-%, besonders bevorzugt mindesten 95 Gew.-%, ganz besonders bevorzugt mindestens 98 Gew.-%.

Der Anteil an Partikeln mit einer Partikelgröße von höchstens 600 μm, beträgt vorzugsweise mindestens 90 Gew.-%, besonders bevorzugt mindesten 95 Gew.-%, ganz besonders bevorzugt mindestens 98 Gew.-%.

Polymerpartikel mit zu großer Partikelgröße senken die Anquellgeschwindigkeit. Daher sollte der Anteil zu großer Polymerpartikel ebenfalls niedrig sein.

Zu große Polymerpartikel werden daher üblicherweise abgetrennt und in die Mahlung des getrockneten Polymergels rückgeführt.

Die Polymerpartikel können zur weiteren Verbesserung der Eigenschaften oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder ß-Hydroxyalkylamide, wie in DE 102 04 938 A1 und US 6,239,230 beschrieben.

Des Weiteren sind in DE 40 20 780 C1 zyklische Karbonate, in DE 198 07 502 A1 2-Oxazolidinon und dessen Derivate, wie 2-Hydroxyethyl-2-oxazolidinon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidinone, in DE 102 04 937 A1 zyklische Harnstoffe, in DE 103 34 584 A1 bizyklische Amidoacetale, in EP 1 199 327 A2 Oxetane und zyklische Harnstoffe und in WO 2003/031482 A1 Morpholin-2,3-dion und dessen Derivate als geeignete Oberflächennachvernetzer beschrieben.

Bevorzugte Oberflächennachvernetzer sind Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyl-2-oxazolidinon, 2-Oxazolidinon und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben

Die Menge an Oberflächennachvernetzer beträgt vorzugsweise 0,001 bis 5 Gew.-%, besonders bevorzugt 0,02 bis 2 Gew.-%, ganz besonders bevorzugt 0,05 bis 1 Gew.-%, jeweils bezogen auf die Polymerpartikel.

In einer bevorzugten Ausführungsform der vorliegenden Erfindung werden vor, während oder nach der Oberflächennachvernetzung zusätzlich zu den Oberflächennachvernetzern polyvalente Kationen auf die Partikeloberfläche aufgebracht.

Die im erfindungsgemäßen Verfahren einsetzbaren polyvalenten Kationen sind beispielsweise zweiwertige Kationen, wie die Kationen von Zink, Magnesium, Kalzium, Eisen und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Hydroxid, Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat, Citrat und Lactat, möglich. Es sind auch Salze mit unterschiedlichen Gegenionen möglich, beispielsweise basische Aluminiumsalze, wie Aluminiummonoacetat oder Aluminiummonolaktat. Aluminiumsulfat, Aluminiummonoacetat und Aluminiumlaktat sind bevorzugt. Außer Metallsalzen können auch Polyamine als polyvalente Kationen eingesetzt werden.

Die Einsatzmenge an polyvalentem Kation beträgt beispielsweise 0,001 bis 1 Gew.-%, vorzugsweise 0,005 bis 0,5 Gew.-%, besonders bevorzugt 0,02 bis 0,2 Gew.-%. jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel thermisch getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; Deutschland), Vrieco-Nauta Continuous Mixer (Hosokawa Micron BV; Doetinchem; Niederlande), Processall Mixmill Mixer (Processall Incorporated; Cincinnati; USA) und Schugi Flexomix® (Hosokawa Micron BV; Doetinchem; Niederlande). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; Deutschland), Hosokawa Bepex® Disc Dryer (Hosokawa Micron GmbH; Leingarten; Deutschland), Holo-Flite® dryers (Metso Minerals Industries Inc.; Danville; USA) und Nara Paddle Dryer (NARA Machinery Europe; Frechen; Deutschland). Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Trocknungstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten.

In einer bevorzugten Ausführungsform der vorliegenden Erfindung werden die wasserabsorbierenden Polymerpartikel nach der thermischen Trocknung gekühlt. Die Kühlung wird vorzugsweise in Kontaktkühlern, besonders bevorzugt Schaufelkühlern, ganz besonders bevorzugt Scheibenkühlern, durchgeführt. Geeignete Kühler sind beispielsweise Hosokawa Bepex® Horizontal Paddle Cooler (Hosokawa Micron GmbH; Leingarten; Deutschland), Hosokawa Bepex® Disc Cooler (Hosokawa Micron GmbH; Leingarten; Deutschland), Holo-Flite® coolers (Metso Minerals Industries Inc.; Danville; USA) und Nara Paddle Cooler (NARA Machinery Europe; Frechen; Deutschland). Überdies können auch Wirbelschichtkühler eingesetzt werden.

Im Kühler werden die wasserabsorbierenden Polymerpartikel auf 20 bis 150°C, vorzugsweise 30 bis 120°C, besonders bevorzugt 40 bis 100°C, ganz besonders bevorzugt 50 bis 80°C, abgekühlt.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nachbefeuchtet werden.

Die Nachbefeuchtung wird vorzugsweise bei 30 bis 80°C, besonders bevorzugt bei 35 bis 70°C, ganz besonders bevorzugt bei 40 bis 60°C, durchgeführt. Bei zu niedrigen Temperaturen neigen die wasserabsorbierenden Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%, jeweils bezogen auf die wasserabsorbierenden Polymerpartikel. Durch die Nachbefeuchtung wird die mechanische Stabilität der Polymerpartikel erhöht und deren Neigung zur statischen Aufladung vermindert. Vorteilhaft wird die Nachbefeuchtung im Kühler nach der thermischen Trocknung durchgeführt.

Geeignete Beschichtungen zur Verbesserung der Anquellgeschwindigkeit sowie der Permeabilität (SFC) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20.

Ein weiterer Gegenstand der vorliegenden Anmeldung ist die aus der Dehydratisierung abgetrennte wässrige Acrylsäure.

Die erfindungsgemäße wässrige Acrylsäure enthält vorzugweise Wasser und

20 bis 80 Gew.-% Acrylsäure,

0,001 bis 1 Gew.-% Ameisensäure,

0,001 bis 1 Gew.-% Essigsäure,

0,001 bis 1 Gew.-% Glykolsäure,

0,0001 bis 0,05 Gew.-% Formaldehyd,

0,0001 bis 0,05 Gew.-% Acetaldehyd,

0,0001 bis 0,05 Gew.-% Methanol,

0,0001 bis 0,05 Gew.-% Ethanol und

0,0001 bis 0,1 Gew.-% eines inerten organischen Lösungsmittels 1.

Die erfindungsgemäße wässrige Acrylsäure enthält besonders bevorzugt Wasser und

30 bis 70 Gew.-% Acrylsäure,

0,005 bis 0,5 Gew.-% Ameisensäure,

0,005 bis 0,5 Gew.-% Essigsäure,

0,005 bis 0,5 Gew.-% Glykolsäure,

0,0002 bis 0,02 Gew.-% Formaldehyd,

0,0002 bis 0,02 Gew.-% Acetaldehyd,

0,0002 bis 0,02 Gew.-% Methanol,

0,0002 bis 0,02 Gew.-% Ethanol und

0,0005 bis 0,05 Gew.-% eines inerten organischen Lösungsmittels 1.

Die erfindungsgemäße wässrige Acrylsäure enthält ganz besonders bevorzugt Wasser und

40 bis 60 Gew.-% Acrylsäure,

0,01 bis 0,1 Gew.-% Ameisensäure,

0,01 bis 0,1 Gew.-% Essigsäure,

0,01 bis 0,1 Gew.-% Glykolsäure,

0,0005 bis 0,01 Gew.-% Formaldehyd,

0,0005 bis 0,01 Gew.-% Acetaldehyd,

0,0005 bis 0,01 Gew.-% Methanol,

0,0005 bis 0,01 Gew.-% Ethanol und

0,001 bis 0,02 Gew.-% eines inerten organischen Lösungsmittels 1.

Das inerte organische Lösungsmittel ist vorzugsweise ausgewählt aus Dimethylphthalat, Diethylphthalat, Dimethylisophthalat, Diethylisophthalat, Dimethylterephthalat, Diethylterephthalat, Nonansäure, Dekansäure, Biphenyl und/oder Diphenylether.

Methoden

Bestimmung des Gehalts an 3-Hydroxoypropionsäure und Acrylsäure

Die Gehalte an 3-Hydroxoypropionsäure und Acrylsäure werden durch Umkehrphasenchromatographie mit Ultraviolett-Detektion bestimmt.

Zu Probenvorbereitung werden ca. 100 bis 300 mg Probe in einen 50ml Messkolben eigewogen und mit Eluent A aufgefüllt. Eluent A ist eine Mischung aus 1000 ml Wasser und 1 ml 0,5molarer Schwefelsäure.

Zur Kalibrierung der 3-Hydroxypropionäure werden vier Einwaagen (ca. 280 mg, 180 mg, 90 mg und 60 mg) verwendet, wobei vor dem Auffüllen des 50ml Messkolbens mit ca. 100 µl 25gew.-%iger Schwefelsäure auf einen pH-Wert von 3 bis 4 angesäuert wird (ggf. nachsäuern). Der Kalibrierbereich beträgt 0,1 bis 280 mg/50ml.

Zur Kalibrierung der Acrylsäure werden mindestens zwei Einwaagen auf mindestens sechs Konzentrationen verdünnt. Der Kalibrierbereich beträgt 0,01 bis 0,9 mg/50ml.

Zur Umkehrphasenchromatographie wird eine Trennsäule vom Typ Prontosil 120-3-C18 AQ 3µm, 150x4,6mm (BISCHOFF Analysentechnik und -geräte GmbH, Leonberg, Deutschland) verwendet. Die Temperatur beträgt 25°C das Injektionsvolumen beträgt 50 µl, der Durchfluss beträgt 1,5 ml/min und die Laufzeit beträgt 15 Minuten. Der UV-Detektor wird auf 205 nm eingestellt. Von Anfang bis 8 Minuten wird 100 Gew.-% Eluent A, von 8 bis 11,5 Minuten wird eine Mischung aus 40 Gew.-% Eluent A und 60 Gew.-% Eluent B, von 11,5 bis Ende wird 100 Gew.-% Eluent A verwendet. Eluent B ist Acetonitril.

Bestimmung des Gehalts an oligomerer 3-Hydroxoypropionsäure und oligomerer Acrylsäure

Die Gehalte an oligomerer 3-Hydroxoypropionsäure und oligomerer Acrylsäure werden durch Ionenausschlus-schromatographie mit Brechungsindex-Detektion bestimmt.

Zur Probenvorbereitung werden die zu analysierenden Komponenten mittels einer Festphasenextraktion (solid phase extraction) von der Probenmatrix getrennt. Dazu wird eine SPE-Kartusche vom Typ Bakerband SiOH 6 ml, 1000 mg (J.T.Baker, Avantor Performance Materials, Inc., Center Valley, PA, USA) verwendet. Die SPE-Kartusche wird mit 6 ml Methanol aktiviert und zweimal mit je 6 ml Eluent gespült. Die SPE-Kartusche darf nie trockenlaufen. Anschließend wird die Probe auf die SPE-Kartusche pipettiert und zehnmal mit je 1 ml Eluent in einen 10 ml Messkolben gespült. Die eingesetzte Probenmenge beträgt bei Sumpfproben 65 µl, bei Kopfproben 85 µl und bei Extraktproben 75 µl. Sofern die Proben kein hydrophobes Lösungsmittel (inertes organisches Lösungsmittel 1, inertes organisches Lösungsmittel 2) enthalten, können diese Proben ohne Extraktion aufgespritzt werden, dazu werden 85 µl direkt in 10 ml Eluent gelöst. Als Eluent wird 0,1 Vol.-%ige wässrige Phosphorsäure verwendet.

Zur Ionenausschlusschromatographie werden zwei Trennsäulen vom Typ Shodex RSpak KC-811, 300x8mm (SHOWA DENKO K.K. Shodex (Separation & HPLC) Group, Kawasaki, Japan) hintereinander geschaltet verwendet. Die Temperatur beträgt 40°C, das Injektionsvolumen beträgt 100 µl, der Durchfluss beträgt 1,0 ml/min und die Laufzeit beträgt 45 Minuten. Als Eluent wird 0,1 gew.-%ige wässriger Phosphorsäure verwendet. Der Autosampler wird auf 15°C gekühlt.

Zur Auswertung wird vor dem Integrieren ein Blindwertabzug gemacht. Dazu wird Eluent injiziert und das so erhaltene Chromatogramm vom Probenchromatogramm abgezogen. Die Auswertung erfolgt über Flächenprozent, wobei mittels folgender Formel in Gewichtsprozent umgerechnet wird:

$$Gewichts\%(Oligomer) = \frac{Gewichts\%(Monomer)}{Fl\ddot{a}chen\%(Monomer)} \, x Fl\ddot{a}chen\%(Oligomer)$$

Zur Auswertung der Oligomeren werden jeweils die Gehalte der Dimeren, Trimeren, Tetrameren und Pentameren (d.h. n = 2 bis 5) addiert. Die Retentionszeiten werden durch Injektion von 3-Hydroxypropionsäure und Diacrylsäure kontrolliert.

Beispiele

Beispiel 1

In einem 2l Dreihalskolben mit Doppelmantel und Destillationsaufsatz wurden 1.500 g einer ca. 30 gew.-%igen wässrigen 3-Hydroxypropionsäure vorgelegt, 3 Stunden bei 100 mbar Wasser abdestilliert und der verbliebene Rückstand bei 40 mbar destilliert. Der Doppelmantel wurde mittels Wärmeträgeröls erwärmt. Die Zusammensetzung von Destillat und Destillationsrückstand wurde analysiert.

Tab. 1: Zusammensetzung des Destillats

| Zeit [h] | 3HPA [Gew.-%] | AS [Gew.-%] | Oligo-3HPA [Gew.-%] | Oligo-AS [Gew.-%] |
|---|---|---|---|---|
| 2,9 | 36,5 | 9,3 | 2,1 | 0,0 |
| 5,5 | 38,2 | 19,4 | 6,3 | 1,6 |
| 6,7 | 12,1 | 51,5 | 6,0 | 20,2 |
| 7,6 | 2,2 | 62,1 | 5,8 | 26,8 |
| 8,8 | 0,5 | 57,6 | 0,2 | 41,4 |

Tab. 2: Zusammensetzung des Destillationsrückstands

| Zeit [h] | Temperatur [°C] | 3HPA [Gew.-%] | AS [Gew.-%] | Oligo-3HPA [Gew.-%] | Oligo-AS [Gew.-%] |
|---|---|---|---|---|---|
| 2,9 | 154 | 42,3 | 0,2 | 41,5 | 9,3 |
| 5,5 | 180 | 16,3 | 0,2 | 55,9 | 21,5 |
| 6,7 | 221 | 1,7 | 0,3 | 56,9 | 37,7 |
| 7,6 | 224 | 0,3 | 0,3 | 38,0 | 59,7 |
| 8,8 | 230 | 0,1 | 0,4 | 31,2 | 58,1 |

3HPA            3-Hydroxypropionsäure
AS                Acrylsäure
Oligo-3HPA    oligomere 3-Hydroxypropionsäure
Oligo-AS        oligomere Acrylsäure

Die Ergebnisse zeigen, dass im Destillationsrückstand zunächst 3-Hydroxypropionsäure in oligomere 3-Hydroxypropionsäure umgewandelt wird. Erst danach entsteht nennenswert Acrylsäure und oligomere Acrylsäure. Vermutlich läuft die Dehydratisierung von 3-Hydroxypropionsäure über oligomere 3-Hydroxypropionsäure als Zwischenstufe. Für eine hohe Ausbeute und eine hohe Selektivität muss daher 3-Hydroxypropionsäure in oligomere 3-Hydroxypropionsäure umgewandelt werden. Somit sind für die Dehydratisierung ausreichende Verweilzeiten notwendig.

Beispiel 2

Die Dehydratisierung (1) wurde in einem Reaktor mit Zwangsumlaufentspannungsverdampfer und aufgesetzter Rektifikationskolonne 1 durchgeführt.

Als Reaktor wurde ein 3l Glasbehälter mit Doppelmantel eingesetzt. Die Flüssigkeitsmenge im Reaktor betrug ca. 2500 g. Dies entspricht einer Verweilzeit von ca. 5 Stunden. Die Temperatur in Reaktor betrug 160°C. Der Reaktor war gleichzeitig der Sumpf der Rektifikationskolonne 1.

Der Zwangsumlaufentspannungsverdampfer bestand aus einer Pumpe, einem Wärmetauscher und einem Druckhalteventil. Der Reaktorinhalt wurde mittels der Pumpe über den Wärmetauscher und das Druckhalteventil im Kreis gefördert. Der Wärmetauscher bestand aus 2 Doppelmantelrohren mit einer Länge von 1000 mm und einer Innenfläche von 0,074 $m^2$.

Die Rektifikationskolonne wurde elektrisch begleitbeheizt und hatte einem Innendurchmesser von 50 mm. Als trennwirksame Einbauten wurde Gewebepackungen vom Typ Rhombopak 9M (Sulzer Chemtech, Winterthur, Schweiz) Die Höhe der Gewebepackungen betrug 500 mm. Der Brüden wurde zweistufig kondensiert und mittels eines Dekanters in eine organische Phase und eine wässrige Phase getrennt.

Die zur Dehydratisierung verwendete wässrige Lösung enthielt
76,5 Gew.-% Wasser,
0,02 Gew.-% Acrylsäure,
0,32 Gew.-% oligomere Acrylsäure,
19,9 Gew.-% 3-Hydroxypropionsäure und
3,24 Gew.-% oligomere 3-Hydroxypropionsäure

Es wurden 500 g/h der wässrigen Lösung und 25 g/h einer Mischung aus 94 Gew.-% Dimethylphthalat und 6 Gew.-% Pentamethyldiethylentriamin auf 48°C vorgewärmt und vor dem Wärmetauscher des Zwangsumlaufentspannungsverdampfers zudosiert.

Durch den Zwangsumlaufentspannungsverdampfer wurden weitere 249 kg/h Reaktorinhalt im Kreis gefördert. Vor dem Druckhalteventil betrug der Druck 1,608 bar und die Temperatur 168°C. Es wurden 27 g/h Reaktorinhalt wurden aus dem Kreis ausgeschleust und verworfen.

Der ausgeschleuste Reaktorinhalt enthielt

1,5 Gew.-% Wasser,

7,2 Gew.-% Acrylsäure,

12,8 Gew.-% oligomere Acrylsäure,

1,1 Gew.-% 3-Hydroxypropionsäure und

8,7 Gew.-% oligomere 3-Hydroxypropionsäure

Die Temperatur am Kopf der Rektifikationskolonne 1 betrug 108°C. Der Brüden wurde in einem ersten Kühler mit 5 kg/h wässriger Phase aus dem Dekanter direkt gekühlt. Das Abgas des ersten Kühlers wurde in einem zweiten Kühler (Nachkühler) indirekt gekühlt. Das Abgas des zweiten Kühlers hatte eine Temperatur von 24°C und einem Druck von 949 mbar.

Aus dem Dekanter wurden 2,0 l/h wässrige Phase als Rücklauf in die Rektifikationskolonne 1 zurückgeführt und 524 g/h als Produkt ausgeschleust. Die organische Phase wurde hinter die Pumpe des Zwangsumlaufentspannungsverdampfers zudosiert.

Die ausgeschleuste wässrige Phase enthielt

76,2 Gew.-% Wasser,

21,8 Gew.-% Acrylsäure,

0,8 Gew.-% oligomere Acrylsäure,

0,1 Gew.-% 3-Hydroxypropionsäure und

1,1 Gew.-% Dimethylphthalat

In den Dekanter wurden 25 g/h einer wässrigen Acrylsäure dosiert. Die wässrige Acrylsäure enthielt 49 Gew.-% Wasser, 49 Gew.-% Acrylsäure, 1 Gew.-% Phenothiazin und 1 Gew.-% Hydrochinonmonomethylether. Die wässrige Acrylsäure enthielt noch ungelöste Anteile, vermutlich Phenothiazin.

Beispiel 3

Die Dehydratisierung (1) wird in einem 10l Glasbehälter mit Doppelmantel durchgeführt. Über einen Bodenablass wird der Inhalt des Glasbehälters mittels einer Pumpe durch einen Rohrbündelwärmetauscher im Kreis gefördert. In dem Glasbehälter werden 4.000 g einer Mischung aus 85 Gew.-% Diphyl und 15 Gew.-% Pentamethyldiethylentriamin vorgelegt. Diphyl ist das eutiktische Gemisch aus Diphenylether und Biphenyl. Der Glasbehälter ist im stationären Zustand zu ca. 80% gefüllt. Unterhalb des Rohrbündelwärmetauschers wird 2 l/h Luft in den Kreis dosiert.

Als Zulauf wird 1.571 g/h wässrige 3-Hydroxypropionsäure, hergestellt gemäß WO 2012/074818 A2, eingesetzt. Die wässrige 3-Hydroxypropionsäure hat folgende Zusammensetzung:

45,0 Gew.-% 3-Hydroxypropionsäure.

12,0 Gew.-% oligomere 3-Hydroxypropionsäure,

40,7 Gew.-% Wasser,

2,0 Gew.-% Acrylsäure,

0,1 Gew.-% oligomere Acrylsäure

0,06 Gew.-% Glykolsäure,

0,05 Gew.-% 2-Hydroxypropionsäure,

0,02 Gew.-% Ameisensäure,

0,02 Gew.-% Essigsäure,

0,02 Gew.-% Bernsteinsäure,

0,02 Gew.-% Fumarsäure,

0,004 Gew.-% Formaldehyd,

0,002 Gew.-% Acetaldehyd,

0,002 Gew.-% Methanol und

0,002 Gew.-% Ethanol

Die Reaktionstemperatur im Glasbehälter beträgt 180°C, der Druck im Glasbehälter beträgt 950 mbar.

Dem Glasbehälter wird 20,8 g/h Rückstand entnommen. Der Rückstand wird mit 25,0 g/h Wasser extrahiert und die organische Phase wird in den Glasbehälter zurückgeführt. Verluste an Diphyl und Pentamethyldiethylentriamin werden regelmäßig ersetzt. Die Zielwerte in der flüssigen Phase für Diphyl und Pentamethyldiethylentriamin betragen ca. 40 Gew.-% und ca. 8 Gew.-%. Die wässrige Phase wird der Extraktion (2) zugeführt.

Auf den Glasbehälter ist eine 25bödige Glockenbodenkolonne mit einem Innendurchmesser von 50mm aufgesetzt. Die Glockenbodenkolonne hat eine elektrische Schutzheizung.

Zwischen dem 20. und 21. Boden der Glockenbodenkolonne ist ein Fangboden eingebaut. Dort wird die Flüssigkeit vollständig entnommen und mittels einer Pumpe durch einen Wärmetauscher gefördert, dabei auf 35°C gekühlt und auf den 25. Boden der Glockenbodenkolonne zurückgeführt. 1.557 g/h der gekühlten Flüssigkeit werden der Extraktion (2) zugeführt. 493 g/h der gekühlten Flüssigkeit werden auf den 20. Boden der Glockenbodenkolonne zurückgeführt. Der Rücklauf wird mit 0,005 Gew.-% Phenothiazin stabilisiert.

Die vom 20. Boden der Glockenbodenkolonne entnommene Flüssigkeit hat folgende Zusammensetzung:

<0,0001 Gew.-% 3-Hydroxypropionsäure.

<0,0001 Gew.-% oligomere 3-Hydroxypropionsäure,

51,2 Gew.-% Wasser,

48,7 Gew.-% Acrylsäure,

<0,0001 Gew.-% oligomere Acrylsäure

0,045 Gew.-% Glykolsäure,

<0,0001 Gew.-% 2-Hydroxypropionsäure,

0,02 Gew.-% Ameisensäure,

0,02 Gew.-% Essigsäure,

<0,0001 Gew.-% Bernsteinsäure,

<0,0001 Gew.-% Fumarsäure,

0,003 Gew.-% Formaldehyd,

<0,0001 Gew.-% Acetaldehyd,

0,001 Gew.-% Methanol,

0,001 Gew.-% Ethanol,

<0,0001 Gew.-% Pentamethyldiethylentriamin und

0,01 Gew.-% Diphyl

Die Extraktion (2) wird in einer 20bödigen Siebbodenkolonne mit einem Innendurchmesser von 50mm durchgeführt. Die wässrigen Phasen aus der Dehydratisierung (1) werden auf 50°C erwärmt und der Siebbodenkolonne am Boden zugeführt. Der Zulauf an wässriger Phase beträgt insgesamt 2.830 g/h. Am Kopf der Siebbodenkolonne werden 2.809 g/h Flüssigkeit aus dem Sumpf der Destillation (3) und 21 g/h Dimethylphthalat mit einer Temperatur von 50°C als Extraktionsmittel zugeführt.

1.024 g/h des am Kopf der Siebbodenkolonne entnommenen wässrigen Extrakts werden am Boden der Siebbodenkolonne zurückgeführt. Das restliche wässrige Extrakt wird verworfen.

Das am Kopf der Siebbodenkolonne entnommene wässrige Extrakt hat folgende Zusammensetzung:

0,02 Gew.-% 3-Hydroxypropionsäure.

0,3 Gew.-% oligomere 3-Hydroxypropionsäure,

96,8 Gew.-% Wasser,

0,6 Gew.-% Acrylsäure,

0,5 Gew.-% oligomere Acrylsäure

0,1 Gew.-% Glykolsäure,

0,1 Gew.-% 2-Hydroxypropionsäure,

0,05 Gew.-% Ameisensäure,

0,025 Gew.-% Essigsäure,

0,05 Gew.-% Bernsteinsäure,

0,05 Gew.-% Fumarsäure,

0,0035 Gew.-% Formaldehyd,

<0,0001 Gew.-% Acetaldehyd,

0,001 Gew.-% Methanol,

0,0003 Gew.-% Ethanol,

0,2 Gew.-% Pentamethyldiethylentriamin,

0,0002 Gew.-% Diphyl und

1,2 Gew.-% Dimethylphthalat

Am Boden der Siebbodenkolonne werden 3.799 g/h organisches Extrakt entnommen und in die Destillation (3) überführt.

Das am Boden der Siebbodenkolonne entnommene organische Extrakt hat folgende Zusammensetzung:

0,002 Gew.-% 3-Hydroxypropionsäure.
0,1 Gew.-% oligomere 3-Hydroxypropionsäure,
3,7 Gew.-% Wasser,
22,2 Gew.-% Acrylsäure,
0,5 Gew.-% oligomere Acrylsäure
0,06 Gew.-% Glykolsäure,
0,035 Gew.-% 2-Hydroxypropionsäure,
0,006 Gew.-% Ameisensäure,
0,02 Gew.-% Essigsäure,
0,005 Gew.-% Bernsteinsäure,
0,008 Gew.-% Fumarsäure,
0,002 Gew.-% Formaldehyd,
0,0005 Gew.-% Acetaldehyd,
0,0005 Gew.-% Methanol,
0,001 Gew.-% Ethanol und
0,06 Gew.-% Pentamethyldiethylentriamin,
1,5 Gew.-% Diphyl und
71,8 Gew.-% Dimethylphthalat

Die Destillation (3) wird in einer 30bödigen Glockenbodenkolonne mit einem Innendurchmesser von 50mm durchgeführt. Die Glockenbodenkolonne hat eine elektrische Schutzheizung.

Der Zulauf der Destillation (3) wird auf 160°C erwärmt und dem 5. Boden der Glockenbodenkolonne zugeführt.

Die Sumpfflüssigkeit der Glockenbodenkolonne wird mittels einer Pumpe durch einen Rohrbündelwärmetauscher im Kreis gefördert. Unterhalb des Rohrbündelwärmetauschers werden 2 l/h Luft in den Kreis dosiert. Die Temperatur und der Druck im Sumpf der Glockenbodenkolonne betragen 220°C bzw. 265 mbar.

Aus dem Sumpf der Glockenbodenkolonne werden 2.809 g/h Flüssigkeit entnommen und in die Extraktion (2) zurückgeführt. Aus dem Sumpf der Glockenbodenkolonne werden weitere 11 g/h Flüssigkeit ausgeschleust und verworfen.

Die aus dem Sumpf der Glockenbodenkolonne entnommene Flüssigkeit hat folgende Zusammensetzung:

0,003 Gew.-% 3-Hydroxypropionsäure.
0,2 Gew.-% oligomere 3-Hydroxypropionsäure,
<0,0001 Gew.-% Wasser,
0,2 Gew.-% Acrylsäure,
0,6 Gew.-% oligomere Acrylsäure
0,07 Gew.-% Glykolsäure,
0,03 Gew.-% 2-Hydroxypropionsäure,
<0,0001 Gew.-% Ameisensäure,
<0,0001 Gew.-% Essigsäure,
0,007 Gew.-% Bernsteinsäure,
0,02 Gew.-% Fumarsäure,
<0,0001 Gew.-% Formaldehyd,
<0,0001 Gew.-% Acetaldehyd,
<0,0001 Gew.-% Methanol,
<0,0001 Gew.-% Ethanol und
0,07 Gew.-% Pentamethyldiethylentriamin,
2,0 Gew.-% Diphyl und
96,8 Gew.-% Dimethylphthalat

Zwischen dem 15. und 16. Boden der Glockenbodenkolonne ist ein Fangboden eingebaut. Dort wird die Flüssigkeit vollständig entnommen und mittels einer Pumpe durch einen Wärmetauscher gefördert, dabei auf 65°C gekühlt und auf den 20. Boden der Glockenbodenkolonne zurückgeführt. 750 g/h der gekühlten Flüssigkeit werden als Rohacrylsäure entnommen. 1.379 g/h der gekühlten Flüssigkeit werden auf den 15. Boden der Glockenbodenkolonne zurückgeführt.

Die Rohacrylsäure hat folgende Zusammensetzung:

<0,0001 Gew.-% 3-Hydroxypropionsäure.

<0,0001 Gew.-% oligomere 3-Hydroxypropionsäure,

3,5 Gew.-% Wasser,

96,4 Gew.-% Acrylsäure,

<0,0001 Gew.-% oligomere Acrylsäure

0,0075 Gew.-% Glykolsäure,

<0,0001 Gew.-% 2-Hydroxypropionsäure,

0,02 Gew.-% Ameisensäure,

0,07 Gew.-% Essigsäure,

<0,0001 Gew.-% Bernsteinsäure,

<0,0001 Gew.-% Fumarsäure,

<0,0001 Gew.-% Formaldehyd,

<0,0001 Gew.-% Acetaldehyd,

0,0005 Gew.-% Methanol,

0,002 Gew.-% Ethanol und

<0,0001 Gew.-% Pentamethyldiethylentriamin,

<0,0001 Gew.-% Diphyl und

<0,0001 Gew.-% Dimethylphthalat

Zwischen dem 25. und 26. Boden der Glockenbodenkolonne ist ein Fangboden eingebaut. Dort wird die Flüssigkeit vollständig entnommen und mittels einer Pumpe durch einen Wärmetauscher gefördert, dabei auf 25°C gekühlt und auf den 30. Boden der Glockenbodenkolonne zurückgeführt. 213 g/h der gekühlten Flüssigkeit werden in die Extraktion (2) zurückgeführt. 161 g/h der gekühlten Flüssigkeit werden auf den 25. Boden der Glockenbodenkolonne zurückgeführt. Der Rücklauf wird mit 0,005 Gew.-% Phenothiazin stabilisiert. Der Druck am Kopf der Glockenbodenkolonne beträgt 100 mbar.

Die vom 25. Boden der Glockenbodenkolonne entnommene Flüssigkeit hat folgende Zusammensetzung:

<0,0001 Gew.-% 3-Hydroxypropionsäure.

<0,0001 Gew.-% oligomere 3-Hydroxypropionsäure,

59,7 Gew.-% Wasser,

40,0 Gew.-% Acrylsäure,

<0,0001 Gew.-% oligomere Acrylsäure

<0,0001 Gew.-% Glykolsäure,

<0,0001 Gew.-% 2-Hydroxypropionsäure,

0,14 Gew.-% Ameisensäure,

0,15 Gew.-% Essigsäure,

<0,0001 Gew.-% Bernsteinsäure,

<0,0001 Gew.-% Fumarsäure,

0,002 Gew.-% Formaldehyd,

0,001 Gew.-% Acetaldehyd,

0,006 Gew.-% Methanol,

0,001 Gew.-% Ethanol und

<0,0001 Gew.-% Pentamethyldiethylentriamin,

<0,0001 Gew.-% Diphyl und

<0,0001 Gew.-% Dimethylphthalat

Das Beispiel zeigt, dass sich die aus nachwachsenden Rohstoffen hergestellte Acrylsäure nach dem erfindungsgemäßen Verfahren auf einfache Weise und mit hoher Ausbeute reinigen lässt.

## Patentansprüche

1. Verfahren zur kontinuierlichen Dehydratisierung von wässriger 3-Hydroxypropionsäure zu wässriger Acrylsäure in flüssiger Phase, wobei die flüssige Phase eine Temperatur von 120 bis 250°C aufweist, wässrige 3-Hydroxypropionsäure kontinuierlich der flüssigen Phase zugeführt wird und die wässrige Acrylsäure kontinuierlich aus der flüssigen Phase entfernt wird, **dadurch gekennzeichnet, dass** die flüssige Phase von 5 bis 95 Gew.-% eines inerten organischen Lösungsmittels 1 enthält und das inerte organische Lösungsmittel 1 bei einem Druck von 1013 mbar (101,3 KPa) einen Siedepunkt von 200 bis 350°C aufweist.

**2.** Verfahren gemäß Anspruch 1, **dadurch gekennzeichnet, dass** die flüssige Phase eine Temperatur von 130 bis 220°C aufweist.

**3.** Verfahren gemäß Anspruch 1 oder 2, **dadurch gekennzeichnet, dass** die flüssige Phase 30 bis 60 Gew.-% eines inerten organischen Lösungsmittels 1 enthält.

**4.** Verfahren gemäß einem der Ansprüche 1 bis 3, **dadurch gekennzeichnet, dass** das inerte organische Lösungsmittel 1 bei einem Druck von 1013 mbar (101,3 KPa) einen Siedepunkt von 280 bis 300°C aufweist.

**5.** Verfahren gemäß einem der Ansprüche 1 bis 4, **dadurch gekennzeichnet, dass** das inerte organische Lösungsmittel 1 ausgewählt ist aus Dimethylphthalat, Diethylphthalat, Dimethylisophthalat, Diethylisophthalat, Dimethylterephthalat, Diethylterephthalat, Nonansäure, Dekansäure, Biphenyl und/oder Diphenylether.

**6.** Verfahren gemäß einem der Ansprüche 1 bis 5, **dadurch gekennzeichnet, dass** die Dehydratisierung basisch oder sauer katalysiert wird.

**7.** Verfahren gemäß einem der Ansprüche 1 bis 6, **dadurch gekennzeichnet, dass** die wässrige Acrylsäure destillativ aus der flüssigen Phase entfernt wird.

**8.** Verfahren gemäß Anspruch 7, **dadurch gekennzeichnet, dass** mit einer Rektifikationskolonne destilliert wird.

**9.** Verfahren gemäß einem der Ansprüche 1 bis 8, **dadurch gekennzeichnet, dass** die Raum-Zeit-Ausbeute von 10 bis 150 kg/h Acrylsäure pro m³ flüssiger Phase beträgt.

**10.** Verfahren gemäß einem der Ansprüche 1 bis 9, **dadurch gekennzeichnet, dass** das Verhältnis von oligomerer 3-Hydroxypropionsäure zu monomerer 3-Hydroxypropionsäure in der wässrigen 3-Hydroxypropionsäure mindestens 1:20 beträgt und/oder das Verhältnis von oligomerer 3-Hydroxypropionsäure zu monomerer 3-Hydroxypropionsäure in der flüssigen Phase mindestens 1:1 beträgt.

**11.** Verfahren gemäß einem der Ansprüche 1 bis 10, **dadurch gekennzeichnet, dass** die Verweilzeit in der flüssigen Phase mindestens 10 Minuten beträgt.

**12.** Verfahren gemäß einem der Ansprüche 1 bis 11, **dadurch gekennzeichnet, dass** der flüssigen Phase ein Polymerisationsinhibitor 1 zugesetzt wird.

**13.** Verfahren gemäß Anspruch 12, **dadurch gekennzeichnet, dass** der Polymerisationsinhibitor 1 ausgewählt ist aus Phenothiazin, Hydrochinon und/oder Hydrochinonmonomethylether.

**14.** Verfahren gemäß einem der Ansprüche 1 bis 13, **dadurch gekennzeichnet, dass** der wässrigen Acrylsäure ein Polymerisationsinhibitor 2 zugesetzt wird.

**15.** Verfahren gemäß Anspruch 14, **dadurch gekennzeichnet, dass** der Polymerisationsinhibitor 2 ausgewählt ist aus Phenothiazin, Hydrochinon und/oder Hydrochinonmonomethylether.

**16.** Wässrige Acrylsäure, enthaltend Wasser und

20 bis 80 Gew.-% Acrylsäure,
0,001 bis 1 Gew.-% Ameisensäure,
0,001 bis 1 Gew.-% Essigsäure,
0,001 bis 1 Gew.-% Glykolsäure,
0,0001 bis 0,05 Gew.-% Formaldehyd,
0,0001 bis 0,05 Gew.-% Acetaldehyd,
0,0001 bis 0,05 Gew.-% Methanol,
0,0001 bis 0,05 Gew.-% Ethanol und
0,0001 bis 0,1 Gew.-% eines inerten organischen Lösungsmittels 1.

**17.** Wässrige Acrylsäure gemäß Anspruch 16, wobei das inerte organische Lösungsmittel 1 ausgewählt ist aus Dimethylphthalat, Diethylphthalat, Dimethylisophthalat, Diethylisophthalat, Dimethylterephthalat, Diethylterephthalat, Nonansäure, Dekansäure, Biphenyl und/oder Diphenylether ist.

## Claims

1. A process for continuously dehydrating aqueous 3-hydroxypropionic acid to aqueous acrylic acid in the liquid phase, the liquid phase having a temperature of 120 to 250°C, aqueous 3-hydroxypropionic acid being continuously supplied to the liquid phase and the aqueous acrylic acid being withdrawn continuously from the liquid phase, wherein the liquid phase comprises 5 to 95% by weight of an inert organic solvent 1 and the inert organic solvent 1 at a pressure of 1013 mbar (101.3 kPa) has a boiling point of 200 to 350°C.

2. The process according to claim 1, wherein the liquid phase has a temperature of 130 to 220°C.

3. The process according to claim 1 or 2, wherein the liquid phase comprises 30 to 60% by weight of an inert organic solvent 1.

4. The process according to any of claims 1 to 3, wherein the inert organic solvent 1 at a pressure of 1013 mbar (101.3 kPa) has a boiling point of 280 to 300°C.

5. The process according to any of claims 1 to 4, wherein the inert organic solvent 1 is selected from dimethyl phthalate, diethyl phthalate, dimethyl isophthalate, diethyl isophthalate, dimethyl terephthalate, diethyl terephthalate, nonanoic acid, decanoic acid, biphenyl and/or diphenyl ether.

6. The process according to any one of claims 1 to 5, wherein the dehydration is base- or acid-catalyzed.

7. The process according to any of claims 1 to 6, wherein the aqueous acrylic acid is removed by distillation from the liquid phase.

8. The process according to claim 7, wherein distillation is effected with a rectification column.

9. The process according to any of claims 1 to 8, wherein the space-time yield is from 10 to 150 kg/h of acrylic acid per $m^3$ of liquid phase.

10. The process according to any of claims 1 to 9, wherein the ratio of oligomeric 3-hydroxypropionic acid to monomeric 3-hydroxypropionic acid in the aqueous 3-hydroxypropionic acid is at least 1:20 and/or the ratio of oligomeric 3-hydroxypropionic acid to monomeric 3-hydroxypropionic acid in the liquid phase is at least 1:1.

11. The process according to any of claims 1 to 10, wherein the residence time in the liquid phase is at least 10 minutes.

12. The process according to any of claims 1 to 11, wherein a polymerization inhibitor 1 is added to the liquid phase.

13. The process according to claim 12, wherein the polymerization inhibitor 1 is selected from phenothiazine, hydroquinone and/or hydroquinone monomethyl ether.

14. The process according to any of claims 1 to 13, wherein a polymerization inhibitor 2 is added to the aqueous acrylic acid.

15. The process according to claim 14, wherein the polymerization inhibitor 2 is selected from phenothiazine, hydroquinone and/or hydroquinone monomethyl ether.

16. An aqueous acrylic acid comprising water and
20 to 80% by weight of acrylic acid,
0.001 to 1% by weight of formic acid,
0.001 to 1% by weight of acetic acid,
0.001 to 1% by weight of glycolic acid,
0.0001 to 0.05% by weight of formaldehyde,
0.0001 to 0.05% by weight of acetaldehyde,
0.0001 to 0.05% by weight of methanol,

0.0001 to 0.05% by weight of ethanol and
0.0001 to 0.1% by weight of an inert organic solvent 1.

17. The aqueous acrylic acid according to claim 16, wherein the inert organic solvent 1 is selected from dimethyl phthalate, diethyl phthalate, dimethyl isophthalate, diethyl isophthalate, dimethyl terephthalate, diethyl terephthalate, nonanoic acid, decanoic acid, biphenyl and/or diphenyl ether.

**Revendications**

1. Procédé pour la déshydratation continue d'acide 3-hydroxypropionique aqueux en acide acrylique aqueux en phase liquide, la phase liquide présentant une température de 120 à 250 °C, l'acide 3-hydroxypropionique aqueux étant alimenté en continu à la phase aqueuse et l'acide acrylique aqueux étant éliminé en continu de la phase aqueuse, **caractérisé en ce que** la phase liquide contient de 5 à 95 % en poids d'un solvant 1 organique inerte et le solvant 1 organique inerte présente un point d'ébullition de 200 à 350 °C à une pression de 1 013 mbars (101,3 kPa).

2. Procédé selon la revendication 1, **caractérisé en ce que** la phase liquide présente une température de 130 à 220 °C.

3. Procédé selon la revendication 1 ou 2, **caractérisé en ce que** la phase liquide contient 30 à 60 % en poids d'un solvant 1 organique inerte.

4. Procédé selon l'une quelconque des revendications 1 à 3, **caractérisé en ce que** le solvant 1 organique inerte présente un point d'ébullition de 280 à 300 °C à une pression de 1 013 mbars (101,3 kPa).

5. Procédé selon l'une quelconque des revendications 1 à 4, **caractérisé en ce que** le solvant 1 organique inerte est choisi parmi le phtalate de diméthyle, le phtalate de diéthyle, l'isophtalate de diméthyle, l'isophtalate de diéthyle, le téréphtalate de diméthyle, le téréphtalate de diéthyle, l'acide nonanoïque, l'acide décanoïque, le biphényle et/ou l'éther de diphényle.

6. Procédé selon l'une quelconque des revendications 1 à 5, **caractérisé en ce que** la déshydratation est catalysée de manière basique ou acide.

7. Procédé selon l'une quelconque des revendications 1 à 6, **caractérisé en ce que** l'acide acrylique aqueux est éliminé de la phase liquide par distillation.

8. Procédé selon la revendication 7, **caractérisé en ce que** la distillation est réalisée avec une colonne de rectification.

9. Procédé selon l'une quelconque des revendications 1 à 8, **caractérisé en ce que** le rendement espace-temps est de 10 à 150 kg/h d'acide acrylique par $m^3$ de phase liquide.

10. Procédé selon l'une quelconque des revendications 1 à 9, **caractérisé en ce que** le rapport d'acide 3-hydroxypropionique oligomérique sur acide 3-hydroxypropionique monomérique dans l'acide 3-hydroxypropionique aqueux est d'au moins 1 : 20 et/ou le rapport d'acide 3-hydroxypropionique oligomérique sur acide 3-hydroxypropionique monomérique dans la phase liquide est d'au moins 1 : 1.

11. Procédé selon l'une quelconque des revendications 1 à 10, caractérisé en ce le temps de séjour dans la phase liquide est d'au moins 10 minutes.

12. Procédé selon l'une quelconque des revendications 1 à 11, **caractérisé en ce qu'**un inhibiteur de polymérisation 1 est ajouté à la phase liquide.

13. Procédé selon la revendication 12, **caractérisé en ce que** l'inhibiteur de polymérisation 1 est choisi parmi la phénothiazine, l'hydroquinone et/ou le monométhyléther d'hydroquinone.

14. Procédé selon l'une quelconque des revendications 1 à 13, **caractérisé en ce qu'**un inhibiteur de polymérisation 2 est ajouté à l'acide acrylique aqueux.

15. Procédé selon la revendication 14, **caractérisé en ce que** l'inhibiteur de polymérisation 2 est choisi parmi la phénothiazine, l'hydroquinone et/ou le monométhyléther d'hydroquinone.

16. Acide acrylique aqueux, contenant de l'eau et
    20 à 80 % en poids d'acide acrylique,
    0,001 à 1 % en poids d'acide formique,
    0,001 à 1 % en poids d'acide acétique,
    0,001 à 1 % en poids d'acide glycolique,
    0,0001 à 0,05 % en poids de formaldéhyde,
    0,0001 à 0,05 % en poids d'acétaldéhyde,
    0,0001 à 0,05 % en poids de méthanol,
    0,0001 à 0,05 % en poids d'éthanol et
    0,0001 à 0,1 % en poids d'un solvant 1 organique inerte.

17. Acide acrylique aqueux selon la revendication 16, le solvant 1 organique inerte étant choisi parmi le phtalate de diméthyle, le phtalate de diéthyle, l'isophtalate de diméthyle, l'isophtalate de diéthyle, le téréphtalate de diméthyle, le téréphtalate de diéthyle, l'acide nonanoïque, l'acide décanoïque, le biphényle et/ou l'éther de diphényle.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2012074818 A2
- US 7538247 B
- WO 2006092271 A2
- WO 2008023039 A1
- JP 2010180171 A
- EP 2565211 A1
- EP 2565212 A1
- WO 2007106099 A
- US 2007219391 A
- WO 2005095320 A
- WO 2012091114 A1
- WO 02090312 A1
- EP 0854129 A1
- EP 0616998 A1
- DE 10039025 A1
- WO 2003041832 A1
- EP 0530438 A1
- EP 0547847 A1
- EP 0559476 A1
- EP 0632068 A1
- WO 9321237 A1
- WO 2003104299 A1
- WO 2003104300 A1
- WO 2003104301 A1
- DE 10331450 A1
- DE 10331456 A1
- DE 10355401 A1
- DE 19543368 A1
- DE 19646484 A1
- WO 9015830 A1
- WO 2002032962 A2
- WO 2001038402 A1
- DE 3825366 A1
- US 6241928 B
- WO 2008040715 A2
- WO 2008052971 A1
- WO 2011026876 A1
- EP 0083022 A2
- EP 0543303 A1
- EP 0937736 A2
- DE 3314019 A1
- DE 3523617 A1
- EP 0450922 A2
- DE 10204938 A1
- US 6239230 B
- DE 4020780 C1
- DE 19807502 A1
- DE 19807992 C1
- DE 19854573 A1
- DE 19854574 A1
- DE 10204937 A1
- DE 10334584 A1
- EP 1199327 A2
- WO 2003031482 A1
- DE 3713601 A1

**In der Beschreibung aufgeführte Nicht-Patentliteratur**

- **F.L. BUCHHOLZ ; A.T. GRAHAM.** Modern Superabsorbent Polymer Technology. Wiley-VCH, 1998, 71-103